# EUROPEAN PATENT APPLICATION

(11) **EP 4 299 122 A2**
(43) Date of publication of application: **03.01.2024**
(21) Application number: 23195334.0
(22) Date of filing: 07.05.2015
(51) Int. Cl.: A61P 17/00

(54) **COMPOSITIONS AND METHODS FOR TREATING SKIN AND MUCOUS MEMBRANE DISEASES**

(30) Priority: 07.05.2014 US 201461989818 P
(62) Divisional of application: 15788711.8
(71) Applicant: The Regents of the University of California, Oakland, CA 94607 (US); The United States Government as represented by the Department of Veterans Affairs, Washington DC 20420 (US)
(72) Inventor: KIM, Jenny, J., Los Angeles, CA 90095-3075 (US); CHAMPER, Jackson, Riverside, CA 92507 (US); YU, Yang, Arcadia, CA 91006 (US)
(74) Representative: Müller-Boré & Partner Patentanwälte PartG mbB

(57) **Abstract**

This invention relates to topical probiotic formulations for use in treating skin conditions.

## Description

### GOVERNMENT SUPPORT

This invention was made with Government support under AR053542, awarded by the National Institutes of Health, and by the U.S. Department of Veterans Affairs. The Government has certain rights in the invention.

### PRIORITY CLAIM

This patent application claims priority to U.S. Provisional Patent Application No. 61/989,818, filed on May 7, 2014, hereby incorporated by reference in its entirety.

### BACKGROUND

Acne affects up to 85% of adolescents and causes significant morbidity. Topical therapy is the first line treatment for mild acne and can be used as adjunctive therapy for moderate to severe acne. However, undesirable side effects such as dryness, redness, and irritation from topical therapies such as tretinoin derivatives and benzoyl peroxide limit patient compliance.

Acne pathogenesis is mediated by several factors including hyperproliferation and abnormal differentiation of keratinocytes, impaction of follicles forming a keratinaceous plug, increased androgens and sebum production, *Propionibacterium acnes* overgrowth, and the production of proinflammatory mediators.

It is known that antimicrobials such as antibiotics can be bactericidal or bacteriostatic, limiting and reducing the quantity of bacteria in our bodies. Retinoids also reduce the amount of bacteria in pilosebacous units and microcomedones. Light based therapies work via photothermal heating, photochemical inactivation of bacteria, and various photoimmunological reactions to improve clinical skin outcomes.

However, despite many commercially available therapies, acne remains a therapeutically challenging condition, with many patients being unresponsive to several attempted therapies, making treatment unpredictable and elusive in many cases.

### SUMMARY

In some aspects, the invention relates to a topical formulation comprising an effective amount of Phylotype III *Propionibacterium acnes.* The formulation may further comprise an effective amount of *Propionibacterium acnes* ribotype 6.

In some aspects, the invention relates to a topical formulation comprising an effective amount of *Propionibacterium acnes* ribotype 6 and an effective amount of either a non-pathogenic and/or low-pathogenic bacteria, fungus, virus, an antimicrobial agent, or a phage.

In some aspects, the invention relates to a method for treating a skin condition in a subject in need thereof, comprising applying to the skin of the subject a topical formulation as described herein.

In some aspects, the invention relates to a method for treating a skin condition in a subject in need thereof, comprising: administering to the subject a composition comprising a therapeutically effective amount of an antimicrobial agent; and applying to the skin of the subject a topical formulation comprising *Propionibacterium acnes* ribotype 6 and/or Phylotype III *Propionibacterium acnes.*

In some aspects, the invention relates to a kit for treating a skin condition, comprising: a composition comprising a therapeutically effective amount of an antimicrobial agent; and a topical formulation comprising a therapeutically effective amount of *Propionibacterium acnes* ribotype 6 and/or a therapeutically effective amount of Phylotype III *Propionibacterium acnes.*

### BRIEF DESCRIPTION OF THE FIGURES

**Figure 1****.** Demonstration of RT6 probiotic use in conjunction with antibiotic treatment. Figure 1 shows the amount of each type of bacterial strain as determined by CFU assay.
**Figure 2****.** Demonstration of phylotype III probiotic use in conjunction with antibiotic treatment. Figure shows amount of each type of bacterial strain as determined by CFU assay.
**Figure 3****.** IL-10 secretion by PBMCs stimulated by different strains of *P. acnes.* II,-10 secretion was measured by ELISA. Representative of three experiments. Statistics show t-test comparison to RT6. *** p<0.001.
**Figure 4****.** IL-10 secretion by PBMCs stimulated by different strains of *P. acnes.* II,-10 secretion was measured by ELISA. Representative of three experiments. Statistics show t-test comparison to Phylotype III. *** p<0.001.
**Figure 5****.** II,-17 secretion by PBMCs stimulated by different strains of *P. acnes.* II,-17 secretion was measured by ELISA. Representative of three experiments. Statistics show t-test comparison to RT6. ** p<0.01, ** p<0.001.

### DETAILED DESCRIPTION

### Overview

Oral probiotics have been studied for treating and/or preventing many diseases, including those of the gastrointestinal tract and even inflammatory skin disorders such as acne vulgaris. Topical probiotics, however, have not been well studied.

Studies have demonstrated that the addition of certain bacterial strains may outcompete and inhibit the growth of skin-related pathogenic bacteria such as *Propionibacterium acnes,* the bacteria most associated with acne, or *Staphylococcus aureus,* which causes many infections of the skin such as impetigo, cellulitis, and others. Specifically, *in vitro* experiments showed that *Lactobacillus casei, Lactobacillus acidophilus, Lactobacillus plantarum, Lactobacillus gasseri,* and *Lactococcus lactis* inhibit growth of *Propionibacterium acnes* on agar plates (Al-Ghazzewi & Tester, Int J Cosmet Sci 32:139-42 (2010)). Another study showed that the presence of *Lactobacillus reuteri* and *Lactobacillus rhamnosus* reduced death of keratinocytes in vitro caused by *Staphylococcus aureus* (Prince et al., Appl Environ Microbiol, 78:5119-26 (2012)). *Propionibacterium acnes* was found to slow growth of *Staphylococcus aureus in vitro* and on mouse skin wounds (Shu et al., PLoS One, 8:55380 (2013)).

The presently-disclosed invention is rooted in the discovery that *Propionibacterium acnes,* the species of bacteria that causes acne, may be used to treat acne by administering non-pathogenic or low-pathogenic strains of *Propionibacterium acnes* to the skin or a mucous membrane to either (1) compete with pathogenic strains, or (2) to repopulate the skin or a mucous membrane following treatment with an antimicrobial agent.

In some aspects, the invention relates to topical formulations comprising bacterial strains. These formulations may be used to treat acne. In preferred embodiments, the bacterial strain is a member of *Propionibacterium acnes* Phylotype III or *Propionibacterium acnes* ribotype 6.

In some aspects, the invention relates to methods of using topical formulations to treat acne. In some embodiments, the method comprises two steps. The first step consists of administering an antimicrobial therapy designed to kill bacteria on the skin and/or on a mucous membrane. In some embodiments, the antimicrobial therapy is the administration of a topical, oral, or injectable antibiotic, a topical antimicrobial agent, or light therapy. The second step consists of administering a topical formulation comprising one or more bacterial strains. Thus, bacteria from the topical formulation repopulate the skin or a mucous membrane following antimicrobial therapy. In some aspects where the antimicrobial therapy is specific and not antimicrobial to the bacteria in the topical formulation, both may be administered simultaneously in a combined formulation.

In some aspects, the invention relates to a kit comprising an antimicrobial agent and a topical formulation, wherein the topical formulation comprises one or more bacterial strains.

### Definitions

As used herein the specification, "a" or "an" may mean one or more. As used herein in the claim(s), when used in conjunction with the word "comprising", the words "a" or "an" may mean one or more than one. As used herein "another" may mean at least a second or more.

The term "low-pathogenic" refers to strains of bacteria, fungus, and viruses that do not produce clinical symptoms in a majority of subjects, including naturally-occurring strains and genetically-modified strains.

The term "non-pathogenic" refers to strains of bacteria, fungus, and viruses that do not produce clinical symptoms in a subject, including naturally-occurring strains and genetically-modified strains. Phylotype III *Propionibacterium acnes* is an example of a non-pathogenic bacteria.

The term "preventing" is art-recognized, and when used in relation to a condition, such as a local recurrence, is well understood in the art, and includes administration of a composition which reduces the frequency of, or delays the onset of, symptoms of a medical condition in a subject relative to a subject which does not receive the composition. Thus, prevention of acne includes, for example, reducing the number of detectable acne lesions in a population of patients receiving a prophylactic treatment relative to an untreated control population, and/or delaying the appearance of detectable lesions in a treated population versus an untreated control population, *e.g.,* by a statistically and/or clinically significant amount.

The term "prophylactic or therapeutic" treatment is art-recognized and includes administration to the host of one or more of the subject compositions. If it is administered prior to clinical manifestation of the unwanted condition (*e.g.,* disease or other unwanted state of the host animal) then the treatment is prophylactic (*i.e.,* it protects the host against developing the unwanted condition), whereas if it is administered after manifestation of the unwanted condition, the treatment is therapeutic (*i.e.,* it is intended to diminish, ameliorate, or stabilize the existing unwanted condition or side effects thereof).

The term "ribotype" refers to strains of *P. acnes.* The ribotyped strains were characterized as in Fitz-Gibbon et al. (J. Investigative Dermatology 133:2152-60 (2013)).

The term "phylotype" refers to strains of *P. acnes.* The phylotyped strains were characterized as in McDowell et al. (PLoS ONE 8(9): e70897 (2013)).

The term "subject" refers to a mammal, including, but not limited to, a human or non-human mammal, such as a bovine, equine, canine, ovine, or feline.

A "therapeutically effective amount" of a compound with respect to the subject method of treatment refers to an amount of the compound(s) in a preparation which, when administered as part of a desired dosage regimen (to a mammal, preferably a human) alleviates a symptom, ameliorates a condition, or slows the onset of disease conditions according to clinically acceptable standards for the disorder or condition to be treated or the cosmetic purpose, *e.g.,* at a reasonable benefit/risk ratio applicable to any medical treatment.

As used herein, the term "treating" or "treatment" includes reversing, reducing, or arresting the symptoms, clinical signs, and underlying pathology of a condition in a manner to improve or stabilize a subject's condition.

### Antimicrobial therapy

It is known that antimicrobials such as antibiotics can be bactericidal or bacteriostatic, limiting and reducing the quantity of bacteria in our bodies. Retinoids also reduce the amount of bacteria in pilosebacous units and microcomedones. Light based therapies work via photothermal heating, photochemical inactivation of bacteria, and various photoimmunological reactions to improve clinical skin outcomes. In general, the above therapies directly act to reduce the amount of pathogenic bacteria in a patient. Thus, the invention proposes that any such therapy that achieves the same goal of reducing the number of pathogenic organisms, when used in combination with subsequent topical probiotic treatment, would lead to replacement of the pathogenic microflora involved in the diseased state with natural microflora enriched in healthy skin or mucous membranes, or less pathogenic species occupying the same ecological niche as the type causing a disease state.

Suitable antibacterial compounds include capreomycins, including capreomycin IA, capreomycin IB, capreomycin IIA and capreomycin IIB; carbomycins, including carbomycin A; carumonam; cefaclor, cefadroxil, cefamandole, cefatrizine, cefazedone, cefazolin, cefbuperazone, cefcapene pivoxil, cefclidin, cefdinir, cefditoren, cefime, ceftamet, cefmenoxime, cefmetzole, cefminox, cefodizime, cefonicid, cefoperazone, ceforanide, cefotaxime, cefotetan, cefotiam, cefoxitin, cefpimizole, cefpiramide, cefpirome, cefprozil, cefroxadine, cefsulodin, ceftazidime, cefteram, ceftezole, ceftibuten, ceftiofur, ceftizoxime, ceftriaxone, cefuroxime, cefuzonam, cephalexin, cephalogycin, cephaloridine, cephalosporin C, cephalothin, cephapirin, cephamycins, such as cephamycin C, cephradine, chlortetracycline; chlarithromycin, clindamycin, clometocillin, clomocycline, cloxacillin, cyclacillin, danofloxacin, demeclocyclin, destomycin A, dicloxacillin, dirithromycin, doxycyclin, epicillin, erythromycin A, ethanbutol, fenbenicillin, flomoxef, florfenicol, floxacillin, flumequine, fortimicin A, fortimicin B, forfomycin, foraltadone, fusidic acid, gentamycin, glyconiazide, guamecycline, hetacillin, idarubicin, imipenem, isepamicin, josamycin, kanamycin, leumycins such as leumycin A1, lincomycin, lomefloxacin, loracarbef, lymecycline, meropenam, metampicillin, methacycline, methicillin, mezlocillin, micronomicin, midecamycins such as midecamycin A1, mikamycin, minocycline, mitomycins such as mitomycin C, moxalactam, mupirocin, nafcillin, netilicin, norcardians such as norcardian A, oleandomycin, oxytetracycline, panipenam, pazufloxacin, penamecillin, penicillins such as penicillin G, penicillin N and penicillin O, penillic acid, pentylpenicillin, peplomycin, phenethicillin, pipacyclin, piperacilin, pirlimycin, pivampicillin, pivcefalexin, porfiromycin, propiallin, quinacillin, ribostamycin, rifabutin, rifamide, rifampin, rifamycin SV, rifapentine, rifaximin, ritipenem, rekitamycin, rolitetracycline, rosaramicin, roxithromycin, sancycline, sisomicin, sparfloxacin, spectinomycin, streptozocin, sulbenicillin, sultamicillin, talampicillin, teicoplanin, temocillin, tetracyclin, thostrepton, tiamulin, ticarcillin, tigemonam, tilmicosin, tobramycin, tropospectromycin, trovafloxacin, tylosin, and vancomycin, and analogs, derivatives, pharmaceutically acceptable salts, esters, prodrugs, and protected forms thereof.

Additionally, the antimicrobial may be a phage. The phage may be P100D, P100A, PAS50, P104A, ATCC 29399B_C, ATCC 29399B_T, PA6_rev, P100.1, P14.4, PAD20, P9.1, P101A, P105, or P1.1. The phage is preferably selected from P1, P9, P14, P100A, P100D, P100.1, P101A, P104A, P105, and ATCC_C, which were characterized in Marinelli et al. (mBio 3: e00279-12 (2012); herein incorporated by reference).

Suitable anti-fungal compounds include ketoconazole, miconazole, fluconazole, clotrimazole, undecylenic acid, sertaconazole, terbinafine, butenafine, clioquinol, haloprogin, nystatin, naftifine, tolnaftate, ciclopirox, amphotericin B, or tea tree oil and analogs, derivatives, pharmaceutically acceptable salts, esters, prodrugs, and protected forms thereof.

Suitable antiviral agents include acyclovir, azidouridine, anismoycin, amantadine, bromovinyldeoxusidine, chlorovinyldeoxusidine, cytarabine, delavirdine, didanosine, deoxynojirimycin, dideoxycytidine, dideoxyinosine, dideoxynucleoside, desciclovir, deoxyacyclovir, efavirenz, enviroxime, fiacitabine, foscamet, fialuridine, fluorothymidine, floxuridine, ganciclovir, hypericin, idoxuridine, interferon, interleukin, isethionate, nevirapine, pentamidine, ribavirin, rimantadine, stavudine, sargramostin, suramin, trichosanthin, tribromothymidine, trichlorothymidine, trifluorothymidine, trisodium phosphomonoformate, vidarabine, zidoviridine, zalcitabine and 3-azido-3-deoxythymidine and analogs, derivatives, pharmaceutically acceptable salts, esters, prodrugs, and protected forms thereof.

Other suitable antiviral agents include 2',3'-dideoxyadenosine (ddA), 2',3'-dideoxyguanosine (ddG), 2',3'-dideoxycytidine (ddC), 2',3'-dideoxythymidine (ddT), 2'3'-dideoxy-dideoxythymidine (d4T), 2'-deoxy-3'-thia-cytosine (3TC or lamivudime), 2',3'-dideoxy-2'-fluoroadenosine, 2',3'-dideoxy-2'-fluoroinosine, 2',3'-dideoxy-2'-fluorothymidine, 2',3'-dideoxy-2'-fluorocytosine, 2'3'-dideoxy-2',3'-didehydro-2'-fluorothymidine (Fd4T), 2'3'-dideoxy-2'-beta-fluoroadenosine (F-ddA), 2'3'-dideoxy-2'-beta-fluoro-inosine (F-ddI), and 2',3'-dideoxy-2'-beta-flurocytosine (F-ddC). In some embodiments, the antiviral agent is selected from trisodium phosphomonoformate, ganciclovir, trifluorothymidine, acyclovir, 3'-azido-3'-thymidine (AZT), dideoxyinosine (ddI), and idoxuridine and analogs, derivatives, pharmaceutically acceptable salts, esters, prodrugs, and protected forms thereof.

### Probiotic composition

In some embodiments, the topical formulation comprises an effective amount of a non-pathogenic and/or low-pathogenic bacteria, fungus, or virus. In some embodiments, the topical formulation comprises a therapeutically effective amount of a non-pathogenic and/or low-pathogenic species of Proteobacteria, Actinobacteria, Firmicutes, *Corynebacterium, Bacillus Bifidobacterium, Janthinobacterium, Lactobacillus, Pseudomonas, Propionibacterium,* and/or *Staphylococcus.* In preferred embodiments, the topical formulation comprises an effective amount of a non-pathogenic and/or low-pathogenic *Bacillus coagulans, Bacillus laterosporus, Bacillus megaterium, Bacillus polymyxa, Bacillus licheniformis, Bacillus pumilus, Bacillus stearothermophilus, Bacillus subtilis, Bacillus uniflagellatus, Bifidobacterium adolescentis, Bifidobacterium animalis, Bifidobacterium bifidum, Bifidobacterium breve, Bifidobacterium infantis, Bifidobacterium lactis, Bifidobacterium longum, Bifidobacterium pseudocatenulatur, Janthinobacterium lividum, Lactobacillus acidophilus, Lactobacillus casei, Lactobacillus fermentum, Lactobacillus gasseri, Lactobacillus johnsonii, Lactobacillus lactis, Lactobacillus plantarum, Lactobacillus paracasei, Lactobacillus reuteri, Lactobacillus rhamnosus,* and/or *Pseudomonas lindbergii.* In more preferred embodiments, the topical formulation comprises an effective amount of Phylotype III *Propionibacterium acnes* and/or an effective amount of *Propionibacterium acnes* ribotype 6.

In some embodiments, the topical formulation comprises 1×10², 5×10², 1×10³, 5×10³, 1×10⁴ 5×10⁴ 1×10⁵ 5×10⁵, 1×10⁶ 5×10⁶ 1×10⁷ 5×10⁷ 1×10⁸ 5×10⁸, 1×10⁹ 5×10⁹, or 1×10¹⁰ bacteria cells per milliliter.

In some embodiments, the invention relates to a topical formulation comprising an effective amount of Phylotype III *Propionibacterium acnes.* In other embodiments, the invention relates to a topical formulation comprising an effective amount of *Propionibacterium acnes* ribotype 6. The topical formulation may comprise an effective amount of Phylotype III *Propionibacterium acnes* and an effective amount of *Propionibacterium acnes* ribotype 6.

The topical formulation may further comprise an effective amount of a non-pathogenic and/or low-pathogenic bacteria, fungus, or virus.

In some embodiments, the topical formulation comprises a therapeutically effective amount of a non-pathogenic and/or low-pathogenic species of Proteobacteria, Actinobacteria, or Firmicutes. In preferred embodiments, the topical formulation comprises a therapeutically effective amount of a non-pathogenic and/or low-pathogenic species of *Corynebacterium, Bacillus Bifidobacterium, Janthinobacterium, Lactobacillus, Pseudomonas, Propionibacterium,* or *Staphylococcus.* In other preferred embodiments, the topical formulation comprises an effective amount of a non-pathogenic and/or low-pathogenic *Bacillus coagulans, Bacillus laterosporus, Bacillus megaterium, Bacillus polymyxa, Bacillus licheniformis, Bacillus pumilus, Bacillus stearothermophilus, Bacillus subtilis, Bacillus uniflagellatus, Bifidobacterium adolescentis, Bifidobacterium animalis, Bifidobacterium bifidum, Bifidobacterium breve, Bifidobacterium infantis, Bifidobacterium lactis, Bifidobacterium longum, Bifidobacterium pseudocatenulatur, Janthinobacterium lividum, Lactobacillus acidophilus, Lactobacillus casei, Lactobacillus fermentum, Lactobacillus gasseri, Lactobacillus johnsonii, Lactobacillus lactis, Lactobacillus plantarum, Lactobacillus paracasei, Lactobacillus reuteri, Lactobacillus rhamnosus,* or *Pseudomonas lindbergii.*

In some embodiments, the topical formulation comprises between approximately 1×10³ and 1×10¹⁰ *Propionibacterium acnes* cells per milliliter. In some preferred embodiments, the topical formulation comprises between approximately 1×10⁴ and 1×10⁸ *Propionibacterium acnes* cells per milliliter. In more preferred embodiments, the topical formulation comprises between approximately 1×10⁵ and 5×10⁷ *Propionibacterium acnes* cells per milliliter.

In some embodiments, the topical formulation comprises an effective amount of a topical retinoid. In some embodiments, the retinoid is retinol, adapalene, tretinoin, or tazarotene.

In some embodiments, the topical formulation comprises an effective amount of a topical antifungal agent. In some embodiments, the topical antifungal agent is ketoconazole, miconazole, fluconazole, clotrimazole, undecylenic acid, sertaconazole, terbinafine, butenafine, clioquinol, haloprogin, nystatin, naftifine, tolnaftate, ciclopirox, amphotericin B, or tea tree oil.

In some embodiments, the topical formulation comprises an effective amount of an antimicrobial agent. In some embodiments, the antimicrobial agent is lindamycin, erythromycin, sulfacetamide sodium/sulfur, silver sulfadiazine, neomycin, polymyxin B, bacitracin, mupirocin, or retapamulin.

In some embodiments, the topical formulation comprises an effective amount of a phage. In some embodiments, the phage is P1, P9, P14, P100A, P100D, P100.1, P101A, P104A, P105, or ATCC_C.

In some embodiments, the topical formulation is an emulsion, cream, lotion, gel, oil, ointment, aerosol spray, or semi-solid formulation. In some embodiments, the topical formulation comprises a carrier, wherein said carrier is selected from the group consisting of trehalose, malto-dextrin, rice flour, micro-crystalline cellulose, magnesium stearate, inositol, fructo-oligosaccharide, gluco-oligosaccharide, dextrose, sucrose, talc, water, physiological salt solution, urea, methanol, ethanol, propanol, butanol, ethylene glycol, propylene glycol, white pertrolatum, isopropyl myristate, lanolin, lanolin alcohol, mineral oil, lavender oil, nasturtium extract oil, sorbitan mono-oleate, cetylstearyl alcohol, hydroxypropyl cellulose, detergent, sucrose stearate, sucrose cocoate, sucrose distearate, 2-ethyl-1,3-hexanediol, polyoxypropylene-15-stearyl ether, glycerol stearate, glycerin, synthetic spermaceti, cetyl alcohol, butylparaben, propylparaben, and methylparaben.

In some embodiments, the invention relates to a method for treating a skin condition in a subject in need thereof, comprising applying to the skin of the subject a topical formulation. In some embodiments, the invention relates to a method for treating a mucous membrane condition in a subject in need thereof, comprising applying to a mucous membrane of the subject a topical formulation.

In some embodiments, the subject is a primate, canine, feline, equine, bovine, ovine, or porcine. In preferred embodiments, the subject is a human. In other preferred embodiments, the subject is a household pet.

In some embodiments, the skin condition is acne, contact dermatitis, actinic dermatitis, dermatitis caused by microbial infection, eczema, or rosacea. In preferred embodiments, the skin condition is acne. In some embodiments, the skin is the skin of the face, scalp, neck, chest, or back. In preferred embodiments, the skin is the skin of the face.

In some embodiments, the method comprises a step of, prior to applying the topical formulation, administering to the subject a composition comprising a therapeutically effective amount of an antimicrobial agent. The antimicrobial agent may be administered topically. The antimicrobial agent can be a benzoyl peroxide, hydrogen peroxide, clindamycin, erythromycin, sulfacetamide sodium/sulfur, silver sulfadiazine, neomycin, polymyxin B, bacitracin, mupirocin, retapamulin, azelaic acid, salicylic acid, or glycolic acid.

Alternatively, the antimicrobial therapy can be an oral antibiotic, injected antibiotic, topical phage therapy, or a light-based therapy, such as photothermal heating or photochemical inactivation of bacteria.

In some embodiments, the invention relates to a kit for treating a skin and/or mucous membrane condition, wherein the kit comprises (1) a composition containing a therapeutically effective amount of an antimicrobial agent and (2) a topical formulation. In some embodiments, the composition is adapted for topical administration. In preferred embodiments, the antimicrobial agent is a phage, benzoyl peroxide, hydrogen peroxide, clindamycin, erythromycin, sulfacetamide sodium/sulfur, silver sulfadiazine, neomycin, polymyxin B, bacitracin, mupirocin, retapamulin, azelaic acid, salicylic acid, or glycolic acid.

In some aspects, the invention relates to a method of preventing or treating a skin condition in a subject comprising administering to the subject an effective amount of any one of the compositions described herein. Administering may comprise topical administration, *i.e.,* administration to the skin.

In some aspects, the invention relates to a method of preventing or treating a mucous membrane condition in a subject comprising administering to the subject an effective amount of any one of the compositions described herein. Administering may comprise topical administration, *i.e.,* administration to the mucous membrane.

### EXEMPLIFICATION

### Example 1: Formulation containing P. acnes ribotype 6

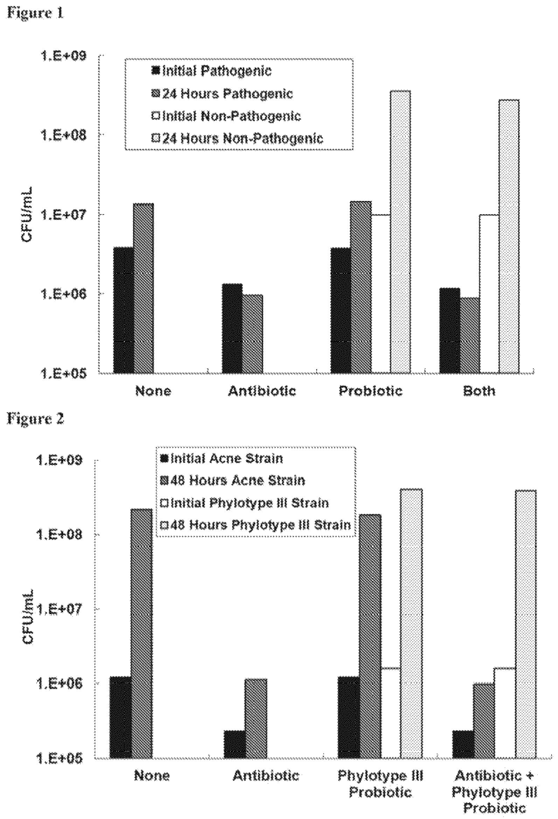

To demonstrate the efficacy of combining the unique formulation of non-pathogenic bacteria in combination with antimicrobial treatment, growth experiments were conducted with *P. acnes* of non-pathogenic ribotype 6 and of acne-associated, pathogenic ribotypes 5 and 8 (Figure 1, Table I). All conditions initially contained a low concentration of acne-associated, pathogenic strains growing in media. Antibiotic treated condition contained sufficient antibiotics to eliminate 90% of bacteria, but as in disease, remaining bacteria were able to grow back. In the probiotic condition, a similar amount of non-pathogenic bacteria were added initially to the media containing the pathogenic strain. The non-pathogenic strains grew faster than the pathogenic strain, and eventually the percentage of pathogenic bacteria was greatly reduced. The amount of pathogenic bacteria was further reduced in the combined treatment, in which the non-pathogenic bacteria were added after antibiotic treatment of the pathogenic strains.

**Table I**

| Demonstration of RT6 topical probiotic use for acne after antibiotic treatment. Table shows % of each type of bacterial strain as determined by CFU assay. | | | | |
|---|---|---|---|---|
| **Treatment** | **Initial Acne %** | **24h Acne %** | **Initial RT6 %** | **24h RT6 %** |
| **None** | 100 | 100 | 0 | 0 |
| **Antibiotic** | 100 | 100 | 0 | 0 |
| **RT6 Probiotic** | 27.0 | 3.9 | 73.0 | 96.1 |
| **Antibiotic + RT6** | 10.5 | 0.3 | 89.5 | 99.7 |

### Example 2: Formulation containing Phylotype III P. acnes

An additional experiment using healthy skin associated Phylotype III *P. acnes* yielded similar results (Figure 2, Table II). The combination of antibiotic treatment followed by probiotic treatment yielded a synergistic improvement because the percentage of pathogenic bacteria in this combination treatment (Tables I and II) was significantly decreased compared to an additive effect of both individual treatments. Lower percentages of pathogenic strains in the total composition of bacterial flora on the skin indicate a reduced likelihood of developing acne in a patient. At 48hrs, the antibiotic treatment alone does not change the percentage of pathogenic strains in the total composition, while the probiotic treatment alone significantly reduces the percentage of pathogenic strains. However, the combination treatment at 48hrs synergistically reduced the percentage of pathogenic bacteria to only 0.3%, a reduction of 1-2 orders of magnitude compared to probiotic alone. Thus, a properly timed combination of these treatments in the clinic (antibiotic followed by probiotic at an appropriate interval) may yield synergistic improvements in the disease state compared to either treatment alone, or compared to an improperly implemented combination of these treatments (both treatments at the same time or probiotic treatment before antibiotic treatment).

**Table II**

| Demonstration of phylotype III topical probiotic use for acne after antibiotic treatment. | | | | |
|---|---|---|---|---|
| Table shows % of each type of bacterial strain as determined by CFU assay. | | | | |
| **Treatment** | **Initial Acne %** | **48h Acne %** | **Initial Type III %** | **48h Type III %** |
| **None** | 100 | 100 | 0 | 0 |
| **Antibiotic** | 100 | 100 | 0 | 0 |
| **Phylotype III Probiotic** | 42.9 | 31.3 | 57.1 | 68.7 |
| **Antibiotic + Phylotype III** | 12.6 | 0.3 | 87.4 | 99.7 |

### Example 3: Analysis of P. acnes strains

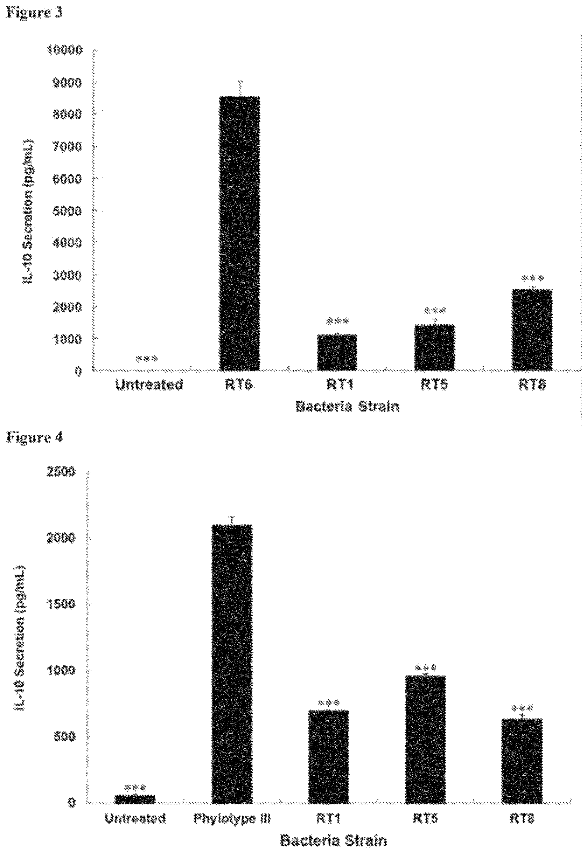

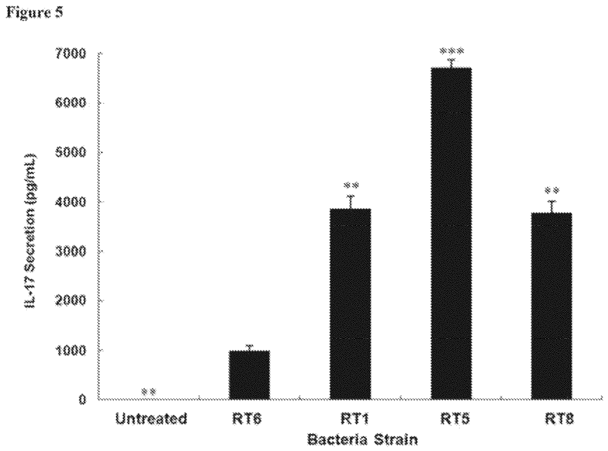

To demonstrate reduced virulence of the strains associated with only healthy skin (ribotype 6 and phylotype III) as compared to acne-associated strains (ribotypes 5 and 8) and strains found in both healthy and acne-affected skin (ribotype 1), peripheral blood mononuclear cells (PBMCs) were stimulated with live *P. acnes.* The ribotype 6 (Figure 3) and phylotype III (Figure 4) strains induced secretion of significantly higher levels of antiinflammatory II,-10 than ribotypes 5, 8, and 1. IL-17 is an inflammatory cytokine thought to play a role in acne pathogenesis. The ribotype 6 strain induced lower IL-17 secretion, while other strains showed significantly higher induction of IL-17 secretion (Figure 5). This indicates that an increase in healthy skin-associated strains compared to acne-associated strains may reduce inflammation in acne, leading to an improvement in the disease state.

### INCORPORATION BY REFERENCE

All of the scientific articles, patents, and published patent applications cited herein are hereby incorporated by reference.

### EQUIVALENTS

Those skilled in the art will recognize, or be able to ascertain using no more than routine experimentation, many equivalents to the specific embodiments of the invention described herein. Such equivalents are intended to be encompassed by the following claims.

The following items are disclosed:
1. A topical formulation comprising an effective amount of Phylotype III *Propionibacterium acnes.*
2. The topical formulation of item 1, further comprising an effective amount of *Propionibacterium acnes* ribotype 6.
3. The topical formulation of item 1 or 2, further comprising an effective amount of a non-pathogenic and/or low-pathogenic bacteria, fungus, or virus.
4. A topical formulation comprising an effective amount of *Propionibacterium acnes* ribotype 6 and an effective amount of either a non-pathogenic and/or low-pathogenic bacteria, fungus, virus, an antimicrobial agent, or a phage.
5. The topical formulation of any one of the preceding items, further comprising a therapeutically effective amount of a non-pathogenic and/or low-pathogenic species of Proteobacteria, Actinobacteria, or Firmicutes.
6. The topical formulation of any one of items 1-4, further comprising a therapeutically effective amount of a non-pathogenic and/or low-pathogenic species of *Corynebacterium, Bacillus Bifidobacterium, Janthinobacterium, Lactobacillus, Pseudomonas, Propionibacterium,* or *Staphylococcus.*
7. The topical formulation of any one of items 1-4, further comprising an effective amount of a non-pathogenic and/or low-pathogenic *Bacillus coagulans, Bacillus laterosporus, Bacillus megaterium, Bacillus polymyxa, Bacillus licheniformis, Bacillus pumilus, Bacillus stearothermophilus, Bacillus subtilis, Bacillus uniflagellatus, Bifidobacterium adolescentis, Bifidobacterium animalis, Bifidobacterium bifidum, Bifidobacterium breve, Bifidobacterium infantis, Bifidobacterium lactis, Bifidobacterium longum, Bifidobacterium pseudocatenulatur, Janthinobacterium lividum, Lactobacillus acidophilus, Lactobacillus casei, Lactobacillus fermentum, Lactobacillus gasseri, Lactobacillus johnsonii, Lactobacillus lactis, Lactobacillus plantarum, Lactobacillus paracasei, Lactobacillus reuteri, Lactobacillus rhamnosus,* or *Pseudomonas lindbergii.*
8. The topical formulation of any one of the preceding items, wherein the formulation comprises between approximately 1×10³ and 1×10¹⁰ *Propionibacterium acnes* cells per milliliter.
9. The topical formulation of item 8, wherein the formulation comprises between approximately 1 ×10⁴ and 1 ×10⁸ *Propionibacterium acnes* cells per milliliter.
10. The topical formulation of item 8, wherein the formulation comprises between approximately 1×10⁵ and 5×10⁷ *Propionibacterium acnes* cells per milliliter.
11. The topical formulation of any one of the preceding items, further comprising an effective amount of a topical retinoid.
12. The topical formulation of item 11, wherein the retinoid is retinol, adapalene, tretinoin, or tazarotene.
13. The topical formulation of any one of the preceding items, further comprising an effective amount of a topical antifungal agent.
14. The topical formulation of item 13, wherein the topical antifungal agent is ketoconazole, miconazole, fluconazole, clotrimazole, undecylenic acid, sertaconazole, terbinafine, butenafine, clioquinol, haloprogin, nystatin, naftifine, tolnaftate, ciclopirox, amphotericin B, or tea tree oil.
15. The topical formulation of any one of the preceding items, further comprising an effective amount of an antimicrobial agent.
16. The topical formulation of item 15, wherein the antimicrobial agent is lindamycin, erythromycin, sulfacetamide sodium/sulfur, silver sulfadiazine, neomycin, polymyxin B, bacitracin, mupirocin, or retapamulin.
17. The topical formulation of any one of the preceding items, further comprising an effective amount of a phage.
18. The topical formulation of item 17, wherein the phage is P1, P9, P14, P100A, P100D, P100.1, P101A, P104A, P105, or ATCC_C.
19. The topical formulation of any one of the preceding items, wherein the formulation is an emulsion, cream, lotion, gel, oil, ointment, aerosol spray, or semi-solid formulation.
20. The topical formulation of any one of the preceding items, further comprising a carrier, wherein said carrier is selected from the group consisting of trehalose, malto-dextrin, rice flour, micro-crystalline cellulose, magnesium stearate, inositol, fructo-oligosaccharide, gluco-oligosaccharide, dextrose, sucrose, talc, water, physiological salt solution, urea, methanol, ethanol, propanol, butanol, ethylene glycol, propylene glycol, white pertrolatum, isopropyl myristate, lanolin, lanolin alcohol, mineral oil, lavender oil, nasturtium extract oil, sorbitan mono-oleate, cetylstearyl alcohol, hydroxypropyl cellulose, detergent, sucrose stearate, sucrose cocoate, sucrose distearate, 2-ethyl-1,3-hexanediol, polyoxypropylene-15-stearyl ether, glycerol stearate, glycerin, synthetic spermaceti, cetyl alcohol, butylparaben, propylparaben, and methylparaben.
21. A method for treating a skin condition in a subject in need thereof, comprising applying to the skin of the subject a topical formulation of any one of the preceding items.
22. The method of item 21, further comprising, prior to applying the topical formulation, administering to the subject a composition comprising a therapeutically effective amount of an antimicrobial agent.
23. A method for treating a skin condition in a subject in need thereof, comprising:
   administering to the subject a composition comprising a therapeutically effective amount of an antimicrobial agent; and
   applying to the skin of the subject a topical formulation comprising *Propionibacterium acnes* ribotype 6 or Phylotype III *Propionibacterium acnes.*
24. The method of item 22 or 23, wherein the antimicrobial agent is administered topically.
25. The method of any one of items 22-24, wherein the antimicrobial agent is a benzoyl peroxide, hydrogen peroxide, clindamycin, erythromycin, sulfacetamide sodium/sulfur, silver sulfadiazine, neomycin, polymyxin B, bacitracin, mupirocin, retapamulin, azelaic acid, salicylic acid, or glycolic acid.
26. The method of item 22 or 23, wherein the antimicrobial agent is an oral antibiotic, injected antibiotic, topical phage therapy, or a light-based therapy, such as photothermal heating or photochemical inactivation of bacteria.
27. The method of any one of items 21-26, wherein the subject is a primate, canine, feline, equine, bovine, ovine, or porcine.
28. The method of any one of items 21-26, wherein the subject is a human.
29. The method of any one of items 21-26, wherein the subject is a household pet.
30. The method of any one of items 21-29, wherein the skin condition is acne, contact dermatitis, actinic dermatitis, dermatitis caused by microbial infection, eczema, or rosacea.
31. The method of any one of items 21-29, wherein the skin condition is acne.
32. The method of any one of items 21-31, wherein the skin is the skin of the face, scalp, neck, chest, or back.
33. The method of item 32, wherein the skin is the skin of the face.
34. A kit for treating a skin condition, comprising:
   a composition comprising a therapeutically effective amount of an antimicrobial agent; and
   a topical formulation of any one of items 1-20.
35. A kit for treating a skin condition, comprising:
   a composition comprising a therapeutically effective amount of an antimicrobial agent; and
   a topical formulation comprising a therapeutically effective amount of *Propionibacterium acnes* ribotype 6 or a therapeutically effective amount of Phylotype III *Propionibacterium acnes.*
36. The kit of item 34 or 35, wherein the composition is adapted for topical administration.
37. The kit of item 34 or 35, wherein the antimicrobial agent is a phage, benzoyl peroxide, hydrogen peroxide, clindamycin, erythromycin, sulfacetamide sodium/sulfur, silver sulfadiazine, neomycin, polymyxin B, bacitracin, mupirocin, retapamulin, azelaic acid, salicylic acid, or glycolic acid.

## Claims

1. A topical formulation for use in inhibiting growth of acne-associated *Propionibacterium acnes* in a subject in need thereof, the topical formulation comprising between 1×10³ and 1×10¹⁰ *Propionibacterium acnes* ribotype 6 cells per millilitre, the use comprising:
applying to the skin of the subject the topical formulation, and
prior to applying the topic formulation, administering to the subject a composition comprising an antimicrobial agent selected from the group consisting of clindamycin, erythromycin, sulfacetamide sodium/sulfur, silver sulfadiazine, neomycin, polymyxin B, bacitracin, mupirocin, and retapamulin.

2. The topical formulation of claim 1, further comprising a non-pathogenic and/or low-pathogenic species of Proteobacteria, Actinobacteria, or Firmicutes, wherein non-pathogenic and/or low-pathogenic species are species that do not produce clinical symptoms in a majority of subjects.

3. The topical formulation of claim 1 or 2, further comprising a non-pathogenic and/or low-pathogenic species of *Corynebacterium, Bacillus Bifidobacterium, Janthinobacterium, Lactobacillus, Pseudomonas, Propionibacterium,* or *Staphylococcus,* wherein low-pathogenic species are species that do not produce clinical symptoms in a majority of subjects.

4. The topical formulation of any one of claims 1-2, further comprising a non-pathogenic and/or low-pathogenic *Bacillus coagulans, Bacillus laterosporus, Bacillus megaterium, Bacillus polymyxa, Bacillus licheniformis, Bacillus pumilus, Bacillus stearothermophilus, Bacillus subtilis, Bacillus uniflagellatus, Bifidobacterium adolescentis, Bifidobacterium animalis, Bifidobacterium bifidum, Bifidobacterium breve, Bifidobacterium infantis, Bifidobacterium lactis, Bifidobacterium longum, Bifidobacterium pseudocatenulatur, Janthinobacterium lividum, Lactobacillus acidophilus, Lactobacillus casei, Lactobacillus fermentum, Lactobacillus gasseri, Lactobacillus johnsonii, Lactobacillus lactis, Lactobacillus plantarum, Lactobacillus paracasei, Lactobacillus reuteri, Lactobacillus rhamnosus,* or *Pseudomonas lindbergii,* wherein low-pathogenic species are species that do not produce clinical symptoms in a majority of subjects.

5. The topical formulation of any one of the preceding claims, further comprising a topical retinoid, such as retinol, adapalene, tretinoin, or tazarotene.

6. The topical formulation of any one of the preceding claims, further comprising a topical antifungal agent, such as ketoconazole, miconazole, fluconazole, clotrimazole, undecylenic acid, sertaconazole, terbinafine, butenafine, clioquinol, haloprogin, nystatin, naftifine, tolnaftate, ciclopirox, amphotericin B, or tea tree oil.

7. The topical formulation of any one of the preceding claims, further comprising a phage, such as P1, P9, P14, P100A, P100D, P100.1, P101A, P104A, P105, or ATCC_C.

8. The topical formulation of any one of the preceding claims, wherein the formulation is an emulsion, cream, lotion, gel, oil, ointment, aerosol spray, or semi-solid formulation.

9. The topical formulation of any one of the preceding claims, further comprising a carrier, wherein said carrier is selected from the group consisting of trehalose, maltodextrin, rice flour, micro-crystalline cellulose, magnesium stearate, inositol, fructo-oligosaccharide, gluco-oligosaccharide, dextrose, sucrose, talc, water, physiological salt solution, urea, methanol, ethanol, propanol, butanol, ethylene glycol, propylene glycol, white petrolatum, isopropyl myristate, lanolin, lanolin alcohol, mineral oil, lavender oil, nasturtium extract oil, sorbitan mono-oleate, cetylstearyl alcohol, hydroxypropyl cellulose, detergent, sucrose stearate, sucrose cocoate, sucrose distearate, 2-ethyl-1,3- hexanediol, polyoxypropylene-15-stearyl ether, glycerol stearate, glycerin, synthetic spermaceti, cetyl alcohol, butylparaben, propylparaben, and methylparaben.

10. A kit for use in a method of inhibiting growth of acne-associated Propionibacterium acnes, comprising:
a composition comprising an antimicrobial agent selected from the group consisting of clindamycin, erythromycin, sulfacetamide sodium/sulfur, silver sulfadiazine,
neomycin, polymyxin B, bacitracin, mupirocin, retapamulin; and a topical formulation of any one of claims 1-9.

11. The kit of claim 10, wherein the composition is adapted for topical administration.
